# EUROPEAN PATENT APPLICATION

(11) **EP 3 659 497 A1**
(43) Date of publication of application: **03.06.2020**
(21) Application number: 18915311.7
(22) Date of filing: 13.07.2018
(51) Int. Cl.: A61B 5/00, A61C 7/00, G16H 50/30

(54) **METHOD OF IDENTIFYING AND PLANNING CORRECTION OF SPECIFIC FEATURES OF HUMAN BODY SYSTEMS ASSOCIATED WITH BITE**

(30) Priority: 16.04.2018 RU 2018113763
(71) Applicant: Dzalaeva, Fatima Kazbekovna, Moscow, 121614 (RU)
(72) Inventor: Dzalaeva, Fatima Kazbekovna, Moscow, 121614 (RU)
(74) Representative: Benatov, Samuil Gabriel
(86) International application number: PCT/RU2018/000463
(87) International publication number: WO 2019/203682

(57) **Abstract**

The invention relates to medicine, and specifically to orthopaedics and orthodontia, and can be used for planning bite correction. A general and dental history is taken. Mental status is determined. The patient's musculoskeletal system is analysed. A polysomnography examination is performed. Body weight and size is measured. Functional diagnostics is performed. All of the resulting data are transferred to a computing unit. When deviations from the norm of values for the respiratory organs and the musculoskeletal system are detected in conjunction with bite problems, pathologies are diagnosed that disrupt organ function in the body systems investigated in a waking state and/or during sleep and associated with bite. A plan of action to correct the identified pathologies is defined, along with bite correction. The method makes it possible to decrease functional problems in the body by virtue of the complex approach to human diagnosis and treatment.

## Description

### FIELD OF THE INVENTION

The invention relates to the field of medicine, namely multidisciplinary dentistry, and represents a complex method of examination, diagnosis, and treatment planning in complex pathologies at the junction of the following fields: dentistry, osteopathy, kinesiology, psychology, speech and language therapy, otolaryngology, neurology, aesthetic medicine, and sports medicine, and can be used for planning of occlusion correction based on the objective findings obtained during the examination of various body systems of a patient (respiratory, central nervous, cardiovascular, musculoskeletal, etc.).

### PRIOR ART

The analysis by Slavicek (R. Slavicek. The Masticatory Organ: Functions and Dysfunctions. M, 2008) is known in the prior art. In it the author defines the masticatory organ as an entirety of the organs belonging to different systems, i.e., the digestive, skeletal, and muscular system. The maxilla and the mandible, TMJ, muscles of mastication, and neck muscles moving the mandible belong to the musculoskeletal system. The teeth, gums, and tongue belong to the digestive system. According to Slavicek's proprietary methodology, functional diagnostics is a set of examination methods that enable detection of the existing pathologies and functional overload, determine the causes and mechanisms behind the development of diseases, and create a plan for solving these problems.

### DISCLOSURE OF THE INVENTION

The object of the invention is a complete and structured sequential examination of patients and collection of primary and secondary documentation for rehabilitation of patients with full arch reconstruction. The problem is solved by supplementing the known method with a screening protocol for sleep disorders (snoring, bruxism, and apnea) and aesthetic and psychological issues, as well as determination of the causal relationship between these issues and malocclusion, and considering these issues during planning of orthopedic and orthodontic treatment.

The technical effect of the invention is a reduction in the functional problems of the body after orthodontic malocclusion correction by means of a complex approach to the diagnostics and treatment in humans and a comprehensive objective functional analysis in various medical fields at the treatment planning stage.

This technical effect is achieved by detecting and planning the correction of the body system characteristics associated with occlusion prior to the beginning of the orthopedic and orthodontic treatment, which includes the stages wherein:
The medical history is collected using the patient's answers to the questionnaire, and the dental history is collected based on an oral examination and the BruxChecker data; the mental status of the patient is determined using the patient's answers to the questionnaire;
The musculoskeletal system of the patient is examined by electromyography, pedobarography, and radiology;
A polysomnography of the patient is performed including blood oxygen saturation analysis, respiratory air flow analysis, EEG, EMG, eye tracking and video recording for tracking the body position during sleep, pulse measurement, as well as instrumental measurement of the size of body parts and body weight of the patient;
Functional diagnostics is performed, including muscle palpation, pedobarography, frontal and lateral teleradiography, orthopantomography, computer tomography of the TMJ, condylography and cephalometry;
Photographs of the patient's face are taken at rest and while smiling, and the parameters of teeth and gums are measured at rest and while smiling;
A diagnostic imaging device is used to obtain the data on the internal structure of the patient's head, the said data are transferred to a computer, processed, and a three-dimensional image of the patient's head containing images of the teeth is displayed on the computer screen, a virtual three-dimensional model of the patient's head is generated, and the obtained image is analyzed by measurements;
The obtained data are analyzed by the computer, where the obtained data are compared with the predefined parameters;
If abnormal parameters of the respiratory organs and the musculoskeletal system combined with occlusion problems are detected, diagnostics of the pathologies interfering with the organ functioning of the examined body systems while awake and/or during sleep and associated with occlusion is performed, and a plan of action is developed to correct the detected pathologies along with occlusion correction.

For implementation of this method, an orthopantomograph, computed tomography (CT) scanner, magnetic resonance imaging (MRI) scanner, or conebeam computerized tomography (CBCT) scanner can be used as the device for diagnostic imaging of the patient's head.

According to the proposed method, photographs of the face and teeth from different angles are taken prior to planning the orthopedic and orthodontic treatment. The obtained photographs are used for an assessment of the aesthetic parameters of the face, lips, teeth, and smile. The smile aesthetics is comprised of a combination of the shape, position, size, and color of the teeth, their proportions and symmetry relative to each other and the facial components. An aesthetic analysis is a complex evaluation of the relationship between all the parameters.

A diagnostic imaging device is used to obtain the data on the internal structure of the patient's head; the said data are transferred to a computer and processed.

The three-dimensional image of the patient's head containing images of the teeth and oral soft tissues is displayed on the computer screen, and a virtual three-dimensional model of the patient's head is generated.

A functional analysis is performed (condylography and cephalometry), and its results are used to determine the centric relation.

The graphical user interface of the computer is used to simulate the desired occlusion in the virtual model of the patient's head by simulating the appearance of the teeth, their position relative to each other, and position of the antagonist teeth.

If pathologies of the respiratory and/or musculoskeletal system affecting the occlusion are detected, remedial measures aimed at correcting these pathologies are taken jointly with the specialists in the related fields, such as psychologists, somnologists, osteopaths, and aestheticians. For example, after a correction of the mandible position, a gap opens in the upper respiratory tract allowing air flow, or pain in the neck and pelvic muscles can be alleviated by correcting the mandible position. The reverse situation is also possible: previous ankle and knee joint injuries may lead to malocclusion, and when an osteopath removes these blocks and compressions, this eliminates the myofascial pain syndrome in the TMJ through an ascending pathway.

### IMPLEMENTATION OF THE INVENTION

The following means are used for the implementation of the proposed method: camera with a video recording feature, myograph, pedobarograph, condylograph, articulator, variator, cephalostat, computer, saddle chair.

This invention employs an improved operating algorithm for the Gamma Dental software designed to simulate occlusion with an addition of somnology, osteopathy, and aesthetic medicine protocols. When planning the occlusion correction, these improvements enable consideration of the functional and aesthetic issues causing or resulting from malocclusion.

The improved software includes several questionnaires, and answers to their questions are transferred to a computer and processed, after which the software displays instructions for further instrumental examinations of the issues detected based on the subjective evidence provided by the patient, in order to determine their relationship with occlusion and to consider these issues during the computer simulation of occlusion.

The patient is surveyed to collect the medical history. The list of questions to be answered by the patient is displayed on the screen via the user interface; the questions require a yes or no answer using checkboxes:
- infections
- urogenital disorders
- cardiovascular disorders
- central nervous system disorders
- respiratory system disorders
- psychological issues
- digestive disorders
- rheumatic disorders
- metabolic disorders
- hormonal disorders
- allergies
- special issues

The dental history is collected. The patient is requested to answer questions from the following list:
- Do you have problems chewing?
- Do you have speech problems?
- Do you have occlusion issues?
- Are your teeth sensitive?
- Do you have problems with opening your mouth very wide?
- Do your jaw joints click? If yes, on which side?
- Do you have jaw joint pain?
- Do you suffer from headaches?
- Do you suffer from seizures or head, neck or throat spasms?
- Do you have dental occlusion issues in general?
- Have you ever been in a serious accident?
- Have you ever received one or more oral intubations?
- Have you ever received orthodontic treatment?
- Have you ever received splint therapy?
- Do you grind your teeth?
- Do you consider treatment necessary?
- Do you think that you have a serious disorder or disease?
- When did you receive your last dental treatment and what was done?
- How would you describe your psychological behavior?

The mental status of the patient is determined using the patient's answers to the questionnaire.

The software also includes a questionnaire for an evaluation on the Epworth Sleepiness Scale. The patient is asked the following question: "How likely are you to doze off or fall asleep in the following situations?" Try not to confuse sleepiness with feeling tired. All situations should refer to your usual way of life in recent times and reflect your most typical behavior. To answer a question, please evaluate your sleepiness on a scale of 0 to 3, where:
0 - would never doze
1 - slight chance of dozing
2 - moderate chance of dozing
3 - high chance of dozing

The following situations are suggested:
- Sitting and reading
- Watching TV
- Sitting, inactive in a public place (e.g., a theatre or a meeting)
- As a passenger in a car for an hour without a break
- Lying down to rest in the afternoon
- Sitting and talking to someone
- Sitting quietly after a lunch (without alcohol)
- In a car, while stopped for a few minutes

Upon completion of the test, the result is provided as a sum of the item scores. A normal result is below 6 (no signs of sleepiness), 6-12 - moderate sleepiness (the patient is referred to a somnologist), 12-17 - significant daytime sleepiness, above 17 - serious health issues, a complex examination by various specialists is required.

Moderate and excessive sleepiness suggests that occlusion issues may affect the sleep quality. For example, malocclusion may result in an insufficient gap for air flow in the upper respiratory tract. The software contains a somnology protocol to determine whether there is a relationship between the sleep issues and occlusion.

The patient is given a questionnaire to evaluate the sleep quality with multiple-choice questions. Question examples:
- Do you snore?
- If yes, your snoring is: (possible snoring volume)
- How often do you snore?
- Does your snoring affect other people?
- Has anyone ever noticed that you had stopped breathing in your sleep?
- How often do you feel tired after sleeping?
- Do you feel increased fatigue while awake?
- Do you fall asleep while driving?
- If yes, how often?
- Do you suffer from high blood pressure?

The answers to these questions are transferred to the computer and are saved in the somnology protocol.

Then, to determine whether the sleep disorder (snoring, apnea, insomnia, etc.) is associated with malocclusion, CNS disorders, or other physiological issues, a polysomnography is performed during sleep and includes a blood oxygen saturation analysis, respiratory air flow analysis, EEG, EMG, eye tracking and video recording for tracking the body position during sleep, pulse measurement, as well as instrumental measurement of the size of body parts and body weight of the patient, in order to determine whether snoring is related to excessive weight.

The obtained data are also saved in the somnology protocol.

The muscles and nerve endings are analyzed by electromyography.

Pedobarography is performed using a pedobarograph.

Photographs of the face and teeth are taken (40 images and 3 speech videos are required for the protocol).

These photographs are used for an analysis of the aesthetic parameters of the face, lips, and teeth, an analysis of the smile aesthetic parameters, and red aesthetic analysis.

The smile aesthetics is comprised of a combination of the shape, position, size, and color of the teeth, their proportions and symmetry relative to each other and the facial components. An aesthetic analysis is a complex evaluation of the relationship between all the parameters. There are three levels of aesthetics in dentistry: macro-, mini-, and micro-.

The macro-aesthetic level is evaluated by analyzing photographs of the patients and marking certain points thereon. The macro-aesthetic dental design is primarily based on the following parameters:
- facial midline
- ratio between the thirds of the face
- interpupillary distance
- nosolabial angle
- parameters of the line between the tip of the nose and the chin

At the mini-aesthetic level, the aesthetic correlation between the position of the lips, gums, and teeth at rest and while smiling is considered. The mini-level must be evaluated from 1.5-0.5 m; a larger distance may result in a faulty assessment. The main aesthetic principles at this level are based on the relationship between the lips, gums, and teeth, and specific features of this relationship are reflected in the analysis of the frontal, transverse, and vertical view of the smile area. Photographs of the patient are used as the main clinical tool in this case. An analysis of this area is performed in the M and E position, at rest, and while smiling, respectively. The following parameters are measured and analyzed in the M position: occlusion height, philtrum height, visibility of the upper incisors. In the E position: the smile line, midline characteristics, smile symmetry, buccal corridor parameters, visible dental area in general and visible teeth area, including the smile factor and the lip line.

The attractiveness and aesthetics of the teeth and gums are evaluated at the micro-level. A visual assessment of micro-aesthetics can be performed from less than 35 cm, i.e., approximately at the distance for putting on make-up. A clinical analysis of the micro-aesthetic level is performed in the oral cavity, for which magnifying instruments and lighting devices are required. The following must be characterized at this level: tooth size, conformance of the values with the golden ratio, axial tilt, interdental space size, position of the contact points of the adjacent teeth, teeth shade progression, and enamel microtexture.

The design of the smile is closely associated with the tissues around the teeth. The shape, position, and contour of the gums are important components of the general smile aesthetics. Healthy gums surrounding a tooth are an integral part of the aesthetics as a whole - the so-called "white" and "red" aesthetic components. The following main parameters in this area are the shape and contour of the gums, gums in the interdental spaces, the upper point of the gingival margin, gingival height (level).

The red aesthetics characterizes the parameters of soft tissues surrounding the teeth. Thus, a harmonious smile is achieved when both teeth and gums are in perfect condition:
- Even and symmetrical gingival contour on the central incisors. The gingival contour of the lateral incisors is 1 mm lower than that of the central incisors.
- The gingival contour of the canines is parallel to the line between two papillae.
- Only a small section of the gum is visible when smiling. The gingival contour must be in harmony with the smile line.
- The length of the central incisors and canines must be at least 12 mm. The lateral incisors must be about 1.5 mm shorter.

A deviation from any of these parameters may significantly affect the smile aesthetics as a whole. Thus, the smile aesthetics is a multi-factor problem that must be solved considering all these factors.

Photographs of the oral soft tissues are taken as well. The photographs are taken while seated, with an unfixed head position, with an open mouth and the tongue protruding as far as possible. The photographs of the tonsils, tongue, uvula, and soft palate are entered into the software protocol. The visibility of the soft tissues is classified using the obtained images into the following classes, where the following soft tissues are visible in a particular position:
Class 1 - soft palate, oral pharynx, uvula
Class 2 - soft palate and uvula
Class 3 - soft palate
Class 4 - hard palate only

Radiological examinations (orthopantomography, CT, MRI, or CBCT) are used for imaging of the TMJ structure and teeth. The width of certain areas of the joint space is measured using the obtained lateral images: the size and symmetry of articular heads, the height and inclination of the posterior slope of articular tubercles, the movement amplitude of the articular heads upon transition from the central occlusion to an open mouth are evaluated.

A functional analysis is performed (condylography and cephalometry).

The centric relation is determined.

An ICD-10 diagnosis is made after determining the centric relation. A plan of action to correct the detected pathologies is developed, according to which the future work with preliminary splint therapy is modelled.

The detected pathologies associated with occlusion are corrected jointly with specialists in the body system where the pathology was detected. In case of aesthetic issues, orthodontic treatment is performed jointly with an aesthetician, in musculoskeletal disorders - jointly with an osteopath, and in sleep and psychological disorders -jointly with a somnologist and/or psychologist.

## Claims

1. method for identification and planning the correction of the peculiarities of the body systems associated with occlusion prior to the beginning of orthopedic and orthodontic treatment, including the stages wherein:
the medical history, dental history and sleep history are collected based on the patient's answers to the questionnaire, an oral examination and the BruxChecker data; the mental status of the patient is determined;
photographs of the patient's face are taken while smiling and at rest, as well as with an open mouth and relaxed muscles;
an analysis of the aesthetic parameters of the face, lips, and teeth, analysis of the aesthetic parameters of the smile, analysis of the aesthetic parameters of the gums, and analysis of visibility of the soft tissues with an open mouth and relaxed muscles are performed;
the musculoskeletal system of the patient is examined by electromyography, pedobarography, and radiology;
a polysomnography of the patient is performed including blood oxygen saturation analysis, respiratory air flow analysis, EEG, EMG, eye tracking and video recording for tracking the body position during sleep, pulse measurement, as well as instrumental measurement of the size of body parts and body weight of the patient;
functional diagnostics is performed, including muscle palpation, pedobarography, frontal and lateral teleradiography, orthopantomography, computer tomography of the TMJ, condylography and cephalometry;
a diagnostic imaging device is used to obtain the data on the internal structure of the patient's head, the said data are transferred to a computer, processed, and a three-dimensional image of the patient's head containing images of the teeth is displayed on the computer screen, a virtual three-dimensional model of the patient's head is generated, and the obtained image is analyzed by measurements;
the obtained data are analyzed on a computer, where the obtained data are compared with the predefined normal parameters of the respiratory organs and the musculoskeletal system while awake and/or during sleep;
if abnormal parameters of the respiratory organs and the musculoskeletal system combined with occlusion problems are detected, diagnostics of the pathologies interfering with the organ functioning of the examined body systems while awake and/or during sleep and associated with occlusion is performed, and a plan of action is developed to correct the detected pathologies along with occlusion correction.
